# EUROPEAN PATENT SPECIFICATION

(11) **EP 2 708 245 B1**
(45) Date of publication and mention of the grant of the patent: **20.03.2019**
(21) Application number: 13184368.2
(22) Date of filing: 13.09.2013
(51) Int. Cl.: A61L 24/00, A61L 24/06

(54) **COMPOSITION, REACTION PRODUCT, KIT AND APPLICATOR, ESPECIALLY FOR USE IN MEDICINE**
ZUSAMMENSETZUNG, REAKTIONSPRODUKT, KIT UND APPLIKATOR, INSBESONDERE ZUR VERWENDUNG IN DER MEDIZIN
COMPOSITION, PRODUIT DE RÉACTION, KIT ET APPLICATEUR, SPÉCIALEMENT À UTILISER DANS LE DOMAINE MÉDICAL

(30) Priority: 14.09.2012 DE 102012216387
(43) Date of publication of application: 19.03.2014
(73) Proprietor: AESCULAP AG, 78532 Tuttlingen (DE)
(72) Inventor: HENKE, Matthias, 88518 Herbertingen (DE); SIEDLE, Gabriel, 79117 Freiburg (DE)
(74) Representative: Patentanwälte Ruff, Wilhelm, Beier, Dauster & Partner mbB

(56) References cited:
- WO-A1-92/09651
- FARIBA DEHGHANI ET AL: "Engineering porous scaffolds using gas-based techniques", CURRENT OPINION IN BIOTECHNOLOGY, vol. 22, no. 5, 3 May 2011 (2011-05-03), pages 661-666, XP028303034, ISSN: 0958-1669, DOI: 10.1016/J.COPBIO.2011.04.005 [retrieved on 2011-04-14]

## Description

The present invention relates to a composition, especially for use in medicine, comprising a component a) containing cyanoacrylate monomers and an aqueous component b), to a reaction product obtainable from the composition and also to a kit based on the composition and to an applicator based on the composition.

The use of cyanoacrylate compositions in medical and non-medical fields is known.

In medicine, compositions of this type are notably used for topical applications and for adhesive bonding or gluing of soft- and hard-tissue defects.

DE 10 2005 007 920 B4, for example, discloses an adhesive composition which is flexible, curable at body temperature and comprises at least one cyanoacrylate component and at least one polysaccharide component.

Cyanoacrylate adhesive compositions comprising certain plasticizing fractions as well as various polymerization initiators form the subject-matter of printed publications DE 10 2008 033 378 A1 and EP 2 143 771 A1.

WO 92/09651 A1 describes a cyanoacrylate composition which forms a foam. In addition to an anionic polymerization initiator, the composition contains a liquid foaming agent in the form of an organic solvent.

Conventional gas foaming and dense gas foaming processes can be used for fabrication of porous scaffolds (Fariba Dehghani et al.: "Engineering porous scaffolds using gas-based techniques"; Current Opinion in Biotechnology 2011, 22: 661-666).

However, the use of certain additions, for example polymerization initiators or certain organic solvents, can be medically concerning.

Moreover, the porosities of cured compositions of the type in question will occasionally be insufficient, which can lead to problems, depending on the intended use.

The present invention therefore has for its object to provide a cyanoacrylate composition which avoids inadequacies known from the prior art and is notable in particular for an improved biocompatibility over compositions of the type in question as well as improved porosity and mechanical stability after curing.

This object is achieved according to the present invention by a composition having the features of independent Claim 1, a kit having the features of Claim 13 and also an applicator having the features of Claim14. Preferred embodiments of the composition form the subject-matter of dependent Claims 2 to 12. The wording of all the claims is hereby explicitly incorporated in the content of the present description by reference.

The invention proposes a composition, especially for use in medicine, comprising a component a) containing cyanoacrylate monomers and an aqueous component b).

The composition further comprises an acid and a salt which salt reacts with the acid to release a gas. In other words, a chemical reaction takes place between the acid and the salt accompanied by release of a gas. The composition, apart from water, is free from a polymerization initiator.

It is a particular advantage that the composition is curable by mixing components a) and b), the cure being based on a polymerization, generally an anionic polymerization, of the cyanoacrylate monomers in component a) to form polycyanoacrylate. The polymerization is generally initiated by the water in component b). Concurrently, the gas released by the salt reacting with the acid causes the curing composition to foam up, as a result of which the cured reaction product, which is preferably in the form of a foam, has a porous structure, in particular an open porous structure, which is a particular advantage. Therefore, the salt provided according to the present invention can be thought of as a foaming agent.

It is a particular advantage that the porosity of the reaction product can be such that improved ingrowth of biological, especially human or animal, tissues into the reaction product is obtainable as a result. Furthermore, a porous reaction product opens up the possibility of using the latter as carrier or matrix/depot for cells and/or active ingredients.

The porosity and specifically the shape stability of the reaction product obtainable from the composition can be adjusted specifically to the particular intended purpose via the acid and salt content of the composition.

In addition, the curing time of the composition can be determinatively influenced via the acid and salt content. Short curing times are particularly desirable as regards the management of severely bleeding tissue defects in order to prevent that the composition may be flushed out of the defect region before curing or that an insufficiently cured composition may become dislodged relative to the defect to be repaired.

It is a further advantage that, apart from water, no (additional) polymerization initiator is needed for curing the composition.

According to the present invention, therefore, the composition does not include an additional polymerization initiator, i.e. apart from water, it is free from a polymerization initiator, especially from an organic or inorganic polymerization initiator, preferably free from an anionic polymerization initiator such as, for example, amines, phosphines, phosphites, polyethylene glycol, phenol-formaldehyde resins, organometallic compounds, aminosilanes, carbamate compounds or the like.

A further advantage is that foaming the composition requires no organic solvent, as described in WO 92/09651 A1.

According to the present invention, therefore, it is further preferable for the composition, apart from the cyanoacrylate monomers in component a), to be free from organic solvents, especially solvents which act as foaming agents, for example pentane, cyclopentane, hexane, cyclohexane, heptane, petroleum ether, diethyl ether, halogenated, especially perhalogenated, hydrocarbons, benzene, dimethyl sulphide or the like.

Higher biocompatibility on the part of the composition is achievable as a net result.

In general, the acid and the salt are spatially separate from each other in the composition in order that premature foaming of the composition may be prevented.

In principle, the acid can be present in component a) or in component b) and the salt correspondingly in component b) or in component a).

In a preferred embodiment, however, the acid is present in component a), while correspondingly the salt is preferably present in component b).

The acid can be an organic acid or an inorganic acid.

Preferably, the acid is selected from the group comprising alendronic acid, formic acid, malic acid, ascorbic acid, barbituric acid, boric acid, bromic acid, hydrobromic acid, butyric acid, capric acid, caproic acid, caprylic acid, chlorous acid, chloric acid, citric acid, cyanoacetic acid, hydrogen cyanide, disilicic acid, ellagic acid, acetic acid, disulphuric acid, hydrofluoric acid, uric acid, hypobromous acid, hypochlorous acid, hypoiodous acid, iodic acid, hydroiodic acid, carbonic acid, lauric acid, margaric acid, monosilicic acid, myristic acid, enanthic acid, palmitic acid, pelargonic acid, pentadecanoic acid, perbromic acid, perchloric acid, periodic acid, peroxodisulphuric acid, peroxonitric acid, phosphoric acid, picric acid, propionic acid, squaric acid, nitric acid, nitrous acid, hydrochloric acid, sulphuric acid, hydrogen sulphide, sulphurous acid, stearic acid, hydrazoic acid, styphnic acid, thiosulphuric acid, tridecanoic acid, trisilicic acid, undecanoic acid, valeric acid, vulpinic acid and mixtures thereof.

With a view to medical/medicinal uses of the composition proposed according to the present invention, organic acids, especially higher fatty acids, are particularly preferred. Acids particularly suitable for medical/medicinal uses can therefore be selected for example from the group comprising acetic acid, butyric acid, valeric acid, caproic acid, enanthic acid, caprylic acid, pelargonic acid, capric acid, lauric acid, myristic acid, palmitic acid, margaric acid, stearic acid and mixtures thereof.

In a further embodiment, the proportion in which the acid is present in component a) is in the range from 0.01 wt% to 80 wt%, especially 1 wt% to 30 wt% and preferably 2 wt% to 8 wt%, based on the overall weight of component a).

The salt provided according to the present invention is preferably an inorganic salt.

Preferably, the salt reacts with the acid to release carbon dioxide.

It is further preferable for the salt to be a carbonate and/or bicarbonate salt, especially a metal carbonate and/or metal bicarbonate, preferably an alkali metal carbonate, an alkali metal bicarbonate, an alkaline earth metal carbonate and/or an alkaline earth metal bicarbonate.

It is particularly preferable for the salt to be selected from the group comprising lithium carbonate (Li₂CO₃), sodium carbonate (Na₂CO₃), potassium carbonate (K₂CO₃), lithium bicarbonate (LiHCO₃), sodium bicarbonate (NaHCO₃), potassium bicarbonate (KaHCO₃), magnesium carbonate (MgCO₃), calcium carbonate (CaCO₃), strontium carbonate (SrCO₃), magnesium bicarbonate (Mg(HCO₃)₂), calcium bicarbonate (Ca(HCO₃)₂), strontium bicarbonate (Sr(HCO₃)₂) and mixtures thereof.

Alternatively, the salt can be a sulphite salt. In this case, sulphur dioxide is the gas released by a reaction with the acid.

In a further embodiment, the proportion in which the salt is present in component b) is in the range from 0.01 wt% to 50 wt%, especially 1 wt% to 40 wt% and preferably 5 wt% to 30 wt%, based on the overall weight of component b).

The cyanoacrylate monomers may in principle be selected from the group comprising alkyl 2-cyanoacrylates, alkoxyalkyl 2-cyanoacrylates, lactoyl 2-cyanoacrylates, alkyllactoyl 2-cyanoacrylates, multifunctional cyanoacrylates, especially biscyanoacrylates and/or triscyanoacrylates, and mixtures thereof.
The cyanoacrylate monomers are preferably selected from the group comprising methyl 2-cyanoacrylate, ethyl 2-cyanoacrylate, n-propyl 2-cyanoacrylate, isopropyl 2-cyanoacrylate, n-butyl 2-cyanoacrylate, isobutyl 2-cyanoacrylate such as, for example, 1-butyl 2-cyanoacrylate and/or 2-butyl 2-cyanoacrylate, n-pentyl 2-cyanoacrylate, isopentyl 2-cyanoacrylate such as, for example, 1-pentyl 2-cyanoacrylate, 2-pentyl 2-cyanoacrylate and/or 3-pentyl 2-cyanoacrylate, cyclopentyl 2-cyanoacrylate, n-hexyl 2-cyanoacrylate, isohexyl 2-cyanoacrylate such as, for example, 1-hexyl 2-cyanoacrylate, 2-hexyl 2-cyanoacrylate, 3-hexyl 2-cyanoacrylate and/or 4-hexyl 2-cyanoacrylate, cyclohexyl 2-cyanoacrylate, n-heptyl 2-cyanoacrylate, isoheptyl 2-cyanoacrylate such as, for example, 1-heptyl 2-cyanoacrylate, 2-heptyl 2-cyanoacrylate, 3-heptyl 2-cyanoacrylate and/or 4-heptyl 2-cyanoacrylate, n-octyl 2-cyanoacrylate, isooctyl 2-cyanoacrylate such as, for example, 1-octyl 2-cyanoacrylate, 2-octyl 2-cyanoacrylate, 3-octyl 2-cyanoacrylate and/or 4-octyl 2-cyanoacrylate, n-nonyl 2-cyanoacrylate, isononyl 2-cyanoacrylate, n-decyl 2-cyanoacrylate, isodecyl 2-cyanoacrylate, n-undecyl 2-cyanoacrylate, isoundecyl 2-cyanoacrylate, n-dodecyl 2-cyanoacrylate, isododecyl 2-cyanoacrylate, lactoyl 2-cyanoacrylate, methoxyisopropyl 2-cyanoacrylate, ethoxyethyl 2-cyanoacrylate, isopropoxyethyl 2-cyanoacrylate, 2-butoxyethyl 2-cyanoacrylate, 2-methoxyethyl 2-cyanoacrylate, butyllactoyl cyanoacrylate and mixtures thereof.

The composition, especially component a) and/or component b), in advantageous embodiments can further contain additives such as, for example, plasticizers, stabilizers/free-radical traps, active biological and/or pharmaceutical ingredients such as, in particular, active wound-healing promoter ingredients, hydroxylapatite, tricalcium phosphate, formaldehyde scavengers or the like.

Suitable plasticizers are preferably selected from the group comprising citric esters, glycerol esters, sebacic esters, fatty acid esters, cellulose esters, polyethylene glycol ethers and mixtures thereof, preferably from the group comprising glycerol triacetate, tributyl acetylcitrate, glycerol tripropionate, glycerol tributyrate, tricaproin, trivalerin, tricaprin, isobutyl myristate, ethyl myristate, ethyl stearate, methyl sebacate, ethyl sebacate, ethylcellulose, polyethylene glycol diethers, preferably polyethylene glycol dimethyl ethers, mannitol, ethylene glycol, liquid polyethylene glycols, 1,2-propylene glycol, liquid poly(1,2-propylene glycol)s, 1,3-propylene glycol, liquid poly(1,3-propylene glycol)s, trialkyl citrates, lactic esters, glycolic esters, glycerol, 2-ethyl-2-hydroxymethyl-1,3-propanediol, branched polyols and mixtures thereof.

Suitable stabilizers/free-radical traps can be selected from the group comprising hydroquinone, t-butylhydroxyanisole, 3,5-di-tert-butyl-4-hydroxytoluene, vitamin E, alpha-tocopherol, beta-tocopherol, gamma-tocopherol, delta-tocopherol and mixtures thereof.

Suitable active wound-healing promoters can be, for example, hyaluronic acid and/or zinc compounds, especially zinc oxide and/or zinc hyaluronate.

Examples of suitable formaldehyde scavengers are sulphite salts, bisulphite salts, ammonium sulphite salts and/or urea salts.

The composition, especially component a) and/or component b), may further contain an oligomer and/or a polymer. It is particularly advantageous that the oligomer/polymer can be used to influence properties of the composition such as, for example, the viscosity, the curing/polymerization rate, the mechanical properties, the adherence properties, the resorbability/degradability, the electrical conductivity and/or the pH of the composition in a specific manner. The oligomer/polymer can further also exert an influence on the miscibility of components a) and b). Suitable oligomers/polymers can be selected from the group comprising polycyanoacrylates, polyvinylpyrrolidones, poly(alpha-hydroxy ester)s, polytrimethylene carbonate, poloxamers, polyethylene glycol, polyacrylates, polyvinyl alcohols, polyvinyl acetates, polyethyleneimines, polysaccharides, polysorbates, polyacrylamides, polyacrylic acids, polyalkyl acrylates, for example methyl acrylate, homopolymers thereof, copolymers thereof and mixtures, especially blends, thereof.

Component a) is preferably in liquid form, while component b) is preferably in the form of an aqueous solution.

To prevent premature curing and especially foaming of the composition, advantageous embodiments have components a) and b) spatially separate from each other in the composition.

Preferably, components a) and b) are present in a volume mixing ratio (component a) to component b)) of 1:100 to 100:1, especially 1:10 to 10:1, preferably 1:1.

The composition in advantageous embodiments is curable by mixing component a) and component b), especially by mixing component a) with an acid, preferably with an acid as disclosed in the preceding description, and component b) with a salt, preferably with a salt as disclosed in the preceding description, within from 0.1 s to 100 s, especially 1 s to 30 s, preferably 2 s to 10 s.

The composition proposed according to the present invention is particularly useful for producing a porous, in particular open porous, and preferably shape-stable (dimensionally stable) foamed structure. The foamed structure is useful in principle not only for medical but also non-medical fields of application.

As far as medical applications are concerned, the composition is especially useful for adhesive bonding or gluing of biological tissues, especially soft tissues and/or hard tissues, preferably bone tissues. The composition can further be used for sealing bodily-fluid leaks, especially blood leaks, and/or air leaks, especially lung leaks. The composition can further be used for producing tissue replacement material, especially soft- or hard-tissue replacement material, preferably bone replacement material, and/or for producing a drug delivery carrier and/or a cell carrier. The composition can further be useful for hemostasis, in particular traumatic hemostasis. Specifically, the composition can be useful for closing battlefield injuries, ballistic trauma and combat-related injuries, in particular gun shot wounds. The composition can be further applied as an occlusion composition, in particular for occluding bodily ducts, for example bile ducts.

The composition can further be contemplated for use in sclerotherapy.

For medical applications, the composition is preferably cured/foamed in situ.

As far as non-medical fields of application are concerned, the composition can be used, for example, for adhesive bonding of technical substrates, especially metal, wood and/or plastics substrates. The composition can additionally be used for producing an insulating material.

A further aspect of the invention relates to a kit as defined in claim 13.

The invention finally relates to an applicator as defined in claim 14.

The applicator can be, for example, a two-chamber syringe or a spraying device such as, for example, an atomizer. To mix components a) and b), the applicator conveniently includes a static or dynamic mixer or a spray head.

With regard to further features and advantages of the applicator, especially of the composition, of component a) and/or of component b), the preceding description is hereby similarly incorporated herein in full by reference.

The recited and further advantages of the invention will be apparent, especially also from the hereinbelow description of an exemplary embodiment in conjunction with the dependent claims. Individual features of the invention can be actualized therein on their own or combined with each other. The embodiments described are merely for elucidation and better understanding of the invention and must not be construed as in any way limiting.

### Production Example 1:

A component a) containing n-butyl cyanoacrylate with an acetic acid content of 4 wt%, based on the overall weight of component a), and a component b), in the form of a 17 wt% potassium bicarbonate solution, were exported using a two-chamber syringe fitted with a static mixer via which components a) and b) were mixed with each other during exportation.

This produced an openly porous foamed structure which initially still had tacky and specifically elastic properties, but within 10 seconds after exportation cured to form a shape-stable structure.

### Production Example 2:

A component a) containing n-butyl cyanoacrylate with an acetic acid content of 4 wt%, based on the overall weight of component a), and a tributyl acetylcitrate content of 10 wt%, based on the overall weight of component a), and a component b), in the form of a 17 wt% potassium bicarbonate solution, were exported using a two-chamber syringe fitted with a static mixer via which components a) and b) were mixed with each other during exportation. This produced a porous foamed structure which, compared with the foamed structure obtained according to Example 1, had distinctly increased elasticity.

### Production Example 3:

A component a) containing n-butyl cyanoacrylate with an acetic acid content of 2 wt%, based on the overall weight of component a), and a component b), in the form of a 17 wt% potassium bicarbonate solution, were exported using a two-chamber syringe fitted with a static mixer via which components a) and b) were mixed with each other during exportation.

This produced a foamed structure which, compared with the foamed structure obtained according to Example 1, possessed distinctly reduced porosity.

### Production Example 4:

A component a) containing n-butyl cyanoacrylate with an acetic acid content of 8 wt%, based on the overall weight of component a), and a component b), in the form of a 17 wt% potassium bicarbonate solution, were exported using a two-chamber syringe fitted with a static mixer via which components a) and b) were mixed with each other during exportation.

This produced a foamed structure which, compared with the foamed structure obtained according to Example 1, possessed distinctly higher porosity.

### Production Example 5:

A component a) containing n-butyl cyanoacrylate with an acetic acid content of 4 wt%, based on the overall weight of component a), and an aqueous component b) containing 17 wt% of potassium bicarbonate and 10 wt% of hydroxylapatite particles (each based on the overall weight of component b)), were exported using a two-chamber syringe fitted with a static mixer via which components a) and b) were mixed with each other during exportation.

The composition cured distinctly faster than the composition used according to Example 1.

### Production Example 6:

A component a) containing n-butyl cyanoacrylate with an acetic acid content of 4 wt%, based on the overall weight of component a), and an aqueous component b) containing 17 wt% of potassium bicarbonate and 10 wt% of tricalcium phosphate particles (each based on the overall weight of component b)), were exported using a two-chamber syringe fitted with a static mixer via which components a) and b) were mixed with each other during exportation.

The composition similarly cured distinctly faster than the composition used according to Example 1 to form a foamed structure.

### Production Example 7:

A component a) containing n-butyl cyanoacrylate with an acetic acid content of 4 wt%, based on the overall weight of component a), and a methyl acrylate content of 20 wt%, based on the overall weight of component a), and a component b), in the form of a 17 wt% potassium bicarbonate solution, were exported using a two-chamber syringe fitted with a static mixer via which components a) and b) were mixed with each other during exportation.

The composition likewise cured distinctly faster than the composition used according to Example 1 to form a foamed structure.

### Production examples 8-28:

According to the general prescription as set out in example 1, components a) and components b) as detailed in table 1 below were mixed with each other, thereby furnishing a foam structure as also detailed in table 1 below.

**Table 1**

| **Component A** | | **Component B** | **Foam Structure** |
|---|---|---|---|
| 96 % NBCA | 4 % Acetic acid | 17 % Potassium hydrogen carbonate solution | Open-cell foam; dimensionally stable structure |
| 96 % NBCA | 4 % Phosphoric acid | 17 % Potassium hydrogen carbonate solution | Closed-cell foam; dimensionally stable structure |
| 96 % NBCA | 4 % Hydrochloric acid | 17 % Potassium hydrogen carbonate solution | Porous foam; dimensionally stable structure |
| 99 % NBCA | 1 % Acetic acid | 17 % Potassium hydrogen carbonate solution | Porous foam; dimensionally stable structure |
| 99 % NBCA | 1 % Phosphoric acid | 17 % Potassium hydrogen carbonate solution | Porous foam; dimensionally stable structure |
| 99 % NBCA | 1 % Hydrochloric acid | 17 % Potassium hydrogen carbonate solution | Porous foam; dimensionally stable structure |
| 99 % NBCA | 1 % Myristic acid | 17 % Potassium hydrogen carbonate solution | Closed-cell foam; dimensionally stable structure |
| 96 % NBCA | 4 % Cyanoacetic acid | 17 % Potassium hydrogen carbonate solution | Porous foam; dimensionally stable structure |
| 96 % NBCA | 4 % Cyanoacetic acid | 5 % Sodium carbonate | Porous foam; dimensionally stable structure |
| 96 % NBCA | 4 % Cyanoacetic acid | 17 % Sodium carbonate | Porous foam; dimensionally stable structure |
| 96 % NBCA | 4 % Cyanoacetic acid | 10 % Sodium disulphite | Closed-cell foam of waxy structure |
| 99 % NBCA | 1 % Hydrochloric acid | 10 % Sodium disulphite | Porous foam of waxy structure |
| 99 % NBCA | 1 % Acetic acid | 10 % Sodium disulphite | Closed-cell foam of waxy structure |
| 99 % NBCA | 1 % Phosphoric acid | 10 % Sodium disulphite | Closed-cell foam of waxy structure |
| 99 % NBCA | 1 % Myristic acid | 10 % Sodium disulphite | Porous foam of waxy structure |
| 96 % NBCA | 4 % Acetic acid | 5 % Sodium carbonate | Porous foam |
| 99 % NBCA | 1 % Acetic acid | 8.5 % Sodium hydrogen carbonate | Porous foam |
| 99 % NBCA | 1 % Myristic acid | 8.5 % Sodium hydrogen carbonate | Porous foam |
| 96 % NBCA | 4 % Acetic acid | 8.5 % Sodium hydrogen carbonate | Porous foam obtained after 70 seconds of polymerization time |
| 99 % NBCA | 1 % Acetic acid | 4.25 % Sodium hydrogen carbonate | Porous foam |
| 99 % NBCA | 1 % Myristic acid | 4.25 % Sodium hydrogen carbonate | Porous foam |

## Claims

1. Composition, especially for use in medicine, comprising a component a) containing cyanoacrylate monomers and an aqueous component b), **characterized in that** the composition further comprises an acid and a salt which reacts with the acid to release a gas and that the composition, apart from water, is free from a polymerization initiator.

2. Composition according to Claim 1, **characterized in that** the acid is present in component a).

3. Composition according to Claim 1 or 2, **characterized in that** the salt is present in component b).

4. Composition according to any preceding claim, **characterized in that** the acid is selected from the group comprising alendronic acid, formic acid, malic acid, ascorbic acid, barbituric acid, boric acid, bromic acid, hydrobromic acid, butyric acid, capric acid, caproic acid, caprylic acid, chlorous acid, chloric acid, citric acid, cyanoacetic acid, hydrogen cyanide, disilicic acid, ellagic acid, acetic acid, disulphuric acid, hydrofluoric acid, uric acid, hypobromous acid, hypochlorous acid, hypoiodous acid, iodic acid, hydroiodic acid, carbonic acid, lauric acid, margaric acid, monosilicic acid, myristic acid, enanthic acid, palmitic acid, pelargonic acid, pentadecanoic acid, perbromic acid, perchloric acid, periodic acid, peroxodisulphuric acid, peroxonitric acid, phosphoric acid, picric acid, propionic acid, squaric acid, nitric acid, nitrous acid, hydrochloric acid, sulphuric acid, hydrogen sulphide, sulphurous acid, stearic acid, hydrazoic acid, styphnic acid, thiosulphuric acid, tridecanoic acid, trisilicic acid, undecanoic acid, valeric acid, vulpinic acid and mixtures thereof.

5. Composition according to any preceding claim, **characterized in that** the proportion in which the acid is present in component a) is in the range from 0.01 wt% to 80 wt%, especially 1 wt% to 30 wt% and preferably 2 wt% to 8 wt%, based on the overall weight of component a).

6. Composition according to any preceding claim, **characterized in that** the salt reacts with the acid to release carbon dioxide.

7. Composition according to any preceding claim, **characterized in that** the salt is an inorganic salt, preferably a carbonate and/or bicarbonate salt, more preferably an alkali metal carbonate, an alkali metal bicarbonate, an alkaline earth metal carbonate and/or an alkaline earth metal bicarbonate.

8. Composition according to any preceding claim, **characterized in that** the proportion in which the salt is present in component b) is in the range from 0.01 wt% to 50 wt%, especially 1 wt% to 40 wt% and preferably 5 wt% to 30 wt%, based on the overall weight of component b).

9. Composition according to any preceding claim, **characterized in that** components a) and b) are spatially separate from each other.

10. Composition according to any preceding claim, **characterized in that** component a) and component b) are present in a volume mixing ratio of 1:100 to 100:1, especially 1:10 to 10:1, preferably 1:1.

11. Composition according to any preceding claim, **characterized in that** the composition is curable by mixing an acid component a) and a foaming agent component b) within from 0.1 s to 100 s, especially 1 s to 30 s, preferably 2 s to 10 s.

12. Composition according to any preceding claim, for producing a porous and specifically shape-stable foamed structure.

13. Kit comprising a composition according to any preceding claim 2-12 and comprising spatially separate from each other an acid present in component a) and a salt present in component b) according to any preceding Claim 2 to 12.

14. Applicator comprising a composition according to any preceding claim 2-12 and comprising a first chamber containing an acid component a) and a second chamber containing a salt component b) according to any of Claims 2 to 12.

## Patentansprüche

1. Zusammensetzung, insbesondere zur Verwendung in der Medizin, umfassend eine Komponente a) enthaltend Cyanacrylat-Monomere und eine wässrige Komponente b), **dadurch gekennzeichnet, dass** die Zusammensetzung ferner eine Säure und ein Salz umfasst, welches durch Umsetzung mit der Säure ein Gas freisetzt, und dass die Zusammensetzung, von Wasser abgesehen, frei von einem Polymerisationsinitiator ist.

2. Zusammensetzung nach Anspruch 1, **dadurch gekennzeichnet, dass** die Säure in der Komponente a) enthalten ist.

3. Zusammensetzung nach Anspruch 1 oder 2, **dadurch gekennzeichnet, dass** das Salz in der Komponente b) enthalten ist.

4. Zusammensetzung nach einem der vorhergehenden Ansprüche, **dadurch gekennzeichnet, dass** die Säure ausgewählt ist aus der Gruppe umfassend Alendronsäure, Ameisensäure, Apfelsäure, Ascorbinsäure, Barbitursäure, Borsäure, Bromsäure, Bromwasserstoffsäure, Buttersäure, Caprinsäure, Capronsäure, Caprylsäure, chlorige Säure, Chlorsäure, Citronensäure, Cyanessigsäure, Cyanwasserstoff, Dikieselsäure, Ellagsäure, Essigsäure, Dischwefelsäure, Flusssäure, Harnsäure, hypobromige Säure, hypochlorige Säure, hypoiodige Säure, Iodsäure, Iodwasserstoffsäure, Kohlensäure, Laurinsäure, Margarinsäure, Monokieselsäure, Myristinsäure, Önanthsäure, Palmitinsäure, Pelargonsäure, Pentadecansäure, Perbromsäure, Perchlorsäure, Periodsäure, Peroxodischwefelsäure, Peroxosalpetersäure, Phosphorsäure, Pikrinsäure, Propionsäure, Quadratsäure, Salpetersäure, salpetrige Säure, Salzsäure, Schwefelsäure, Schwefelwasserstoff, schweflige Säure, Stearinsäure, Stickstoffwasserstoffsäure, Styphninsäure, Thioschwefelsäure, Tridecansäure, Trikieselsäure, Undecansäure, Valeriansäure, Vulpinsäure und Mischungen davon.

5. Zusammensetzung nach einem der vorhergehenden Ansprüche, **dadurch gekennzeichnet, dass** die Säure in der Komponente a) in einem Anteil von 0,01 Gew.-% bis 80 Gew.-%, insbesondere 1 Gew.-% bis 30 Gew.-%, vorzugsweise 2 Gew.-% bis 8 Gew.-%, enthalten ist, bezogen auf das Gesamtgewicht der Komponente a).

6. Zusammensetzung nach einem der vorhergehenden Ansprüche, **dadurch gekennzeichnet, dass** das Salz durch Umsetzung mit der Säure Kohlendioxid freisetzt.

7. Zusammensetzung nach einem der vorhergehenden Ansprüche, **dadurch gekennzeichnet, dass** das Salz ein anorganisches Salz, bevorzugt ein Carbonat- und/oder Hydrogencarbonatsalz, besonders bevorzugt ein Alkalimetallcarbonat, ein Alkalimetallhydrogencarbonat, ein Erdalkalimetallcarbonat und/oder ein Erdalkalimetallhydrogencarbonat, ist.

8. Zusammensetzung nach einem der vorhergehenden Ansprüche, **dadurch gekennzeichnet, dass** das Salz in der Komponente b) in einem Anteil von 0,01 Gew.-% bis 50 Gew.-%, insbesondere 1 Gew.-% bis 40 Gew.-%, vorzugsweise 5 Gew.-% bis 30 Gew.-%, enthalten ist, bezogen auf das Gesamtgewicht der Komponente b).

9. Zusammensetzung nach einem der vorhergehenden Ansprüche, **dadurch gekennzeichnet, dass** die Komponenten a) und b) räumlich getrennt voneinander vorliegen.

10. Zusammensetzung nach einem der vorhergehenden Ansprüche, **dadurch gekennzeichnet, dass** die Komponente a) und die Komponente b) in einem Volumen-Mischungsverhältnis von 1:100 bis 100:1, insbesondere 1:10 bis 10:1, bevorzugt 1:1, vorliegen.

11. Zusammensetzung nach einem der vorhergehenden Ansprüche, **dadurch gekennzeichnet, dass** die Zusammensetzung durch Mischen einer säurehaltigen Komponente a) und einer schaumbildnerhaltigen Komponente b) innerhalb von 0,1 s bis 100 s, insbesondere 1 s bis 30 s, bevorzugt 2 s bis 10 s, aushärtbar ist.

12. Zusammensetzung nach einem der vorhergehenden Ansprüche zur Herstellung einer porösen und insbesondere formstabilen Schaumstruktur.

13. Kit umfassend eine Zusammensetzung nach einem der vorhergehenden Ansprüche 2 bis 12 und umfassend räumlich voneinander getrennt eine in Komponente a) vorliegende Säure und ein in Komponente b) vorliegendes Salz nach einem der vorhergehenden Ansprüche 2 bis 12.

14. Austragsvorrichtung umfassend eine Zusammensetzung nach einem der vorhergehenden Ansprüche 2 bis 12 und umfassend eine erste Kammer enthaltend eine säurehaltige Komponente a) und eine zweite Kammer enthaltend eine salzhaltige Komponente b) nach einem der Ansprüche 2 bis 12.

## Revendications

1. Composition, spécialement pour utilisation en médecine, comprenant un composant a) contenant des monomères de cyanoacrylate et un composant aqueux b), **caractérisée en ce que** la composition comprend en outre un acide et un sel qui réagit avec l'acide pour libérer un gaz et **en ce que** la composition est exempte d'initiateur de polymérisation, à l'exception de l'eau.

2. Composition selon la Revendication 1, **caractérisée en ce que** l'acide est présent dans le composant a).

3. Composition selon la Revendication 1 ou 2, **caractérisée en ce que** le sel est présent dans le composant b).

4. Composition selon l'une quelconque des revendications précédentes, **caractérisée en ce que** l'acide est choisi dans le groupe comprenant les suivants : acide alendronique, acide formique, acide malique, acide ascorbique, acide barbiturique, acide borique, acide bromique, acide bromhydrique, acide butyrique, acide caprique, acide caproïque, acide caprylique, acide chloreux, acide chlorique, acide citrique, acide cyanoacétique, cyanure d'hydrogène, acide disilicique, acide ellagique, acide acétique, acide disulfurique, acide fluorhydrique, acide urique, acide hypobromeux, acide hypochloreux, acide hypoïodeux, acide iodique, acide hydroïodique, acide carbonique, acide laurique, acide margarique, acide monosilicique, acide myristique, acide énanthique, acide palmitique, acide pelargonique, acide pentadécanoïque, acide perbromique, acide perchlorique, acide periodique, acide peroxodisulfurique, acide peroxonitrique, acide phosphorique, acide picrique, acide propionique, acide squarique, acide nitrique, acide nitreux, acide chlorhydrique, acide sulfurique, sulfure d'hydrogène, acide sulfureux, acide stéarique, acide hydrazoïque, acide styphnique, acide thiosulfurique, acide tridécanoïque, acide trisilicique, acide undécanoïque, acide valérique, acide vulpinique et leurs mélanges.

5. Composition selon l'une quelconque des revendications précédentes, **caractérisée en ce que** la proportion dans laquelle l'acide est présent dans le composant a) est incluse dans une plage allant de 0,01 % en masse à 80 % en masse, spécialement de 1 % en masse à 30 % en masse et préférentiellement de 2 % en masse à 8 % en masse, en se basant sur la masse globale du composant a).

6. Composition selon l'une quelconque des revendications précédentes, **caractérisée en ce que** le sel réagit avec l'acide pour libérer du dioxyde de carbone.

7. Composition selon l'une quelconque des revendications précédentes, **caractérisée en ce que** le sel est un sel inorganique, préférentiellement un carbonate et/ou un sel de bicarbonate, plus préférentiellement un carbonate de métal alcalin, un bicarbonate de métal alcalin, un carbonate de métal alcalino-terreux et/ou un bicarbonate de métal alcalino-terreux.

8. Composition selon l'une quelconque des revendications précédentes, **caractérisée en ce que** la proportion dans laquelle le sel est présent dans le composant b) est incluse dans une plage allant de 0,01 % en masse à 50 % en masse, spécialement de 1 % en masse à 40 % en masse et préférentiellement de 5 % en masse à 30 % en masse, en se basant sur la masse globale du composant b).

9. Composition selon l'une quelconque des revendications précédentes, **caractérisée en ce que** les composants a) et b) sont spatialement distincts l'un de l'autre.

10. Composition selon l'une quelconque des revendications précédentes, **caractérisée en ce que** le composant a) et le composant b) sont présents dans un rapport de mélangeage en volume de 1:100 à 100:1, spécialement de 1:10 à 10:1, préférentiellement de 1:1.

11. Composition selon l'une quelconque des revendications précédentes, **caractérisée en ce que** la composition est durcissable par mélangeage d'un composant acide a) et d'un composant de type agent d'expansion b) dans une période comprise entre 0,1 s et 100 s, spécialement entre 1 s et 30 s, préférentiellement entre 2 s et 10 s.

12. Composition selon l'une quelconque des revendications précédentes destinée à produire une structure expansée poreuse et spécifiquement de forme stable.

13. Kit comprenant une composition selon l'une quelconque des revendications précédentes 2 à 12 et comprenant, spatialement distincts l'un de l'autre, un acide présent dans le composant a) et un sel présent dans le composant b) selon l'une quelconque des Revendications 2 à 12 précédentes.

14. Applicateur comprenant une composition selon l'une quelconque des revendications 2 à 12 précédentes et comprenant une première chambre contenant un composant acide a) et une deuxième chambre contenant un composant de type sel b) selon l'une quelconque des Revendications 2 à 12.
